# EUROPEAN PATENT APPLICATION

(11) **EP 4 450 608 A1**
(43) Date of publication of application: **23.10.2024**
(21) Application number: 22907401.8
(22) Date of filing: 12.12.2022
(51) Int. Cl.: C12M 3/00

(54) **CELL CULTURE SYSTEM, METHOD FOR USING CELL CULTURE SYSTEM, AND CELL CULTURE UNIT**

(30) Priority: 16.12.2021 JP 2021203852; 03.02.2022 JP 2022015695
(71) Applicant: Toyo Seikan Group Holdings, Ltd., Shinagawa-ku Tokyo 141-8627 (JP)
(72) Inventor: KOSEKI Osamu, Yokohama-shi, Kanagawa 240-0062 (JP); TANAKA Satoshi, Yokohama-shi, Kanagawa 240-0062 (JP); TOTANI Takahiko, Yokohama-shi, Kanagawa 240-0062 (JP); NISHIYAMA Takaharu, Yokohama-shi, Kanagawa 240-0062 (JP)
(74) Representative: Hasegawa, Kan
(86) International application number: PCT/JP2022/045609
(87) International publication number: WO 2023/112877

(57) **Abstract**

Provided is a cell culture system that can culture cells by providing a culture bag, is allowed to be easily transported with various tools necessary for the culture in one package and can utilize the culture bag for observation with a general-purpose microscope. This is a cell culture system for culturing cells using the culture bag made of soft packaging material, comprising a culture unit having a cutout portion on which a bag housing component having a pressing member that presses the culture bag is mounted, wherein a bag placed on the bag housing component, a medium vessel supplying a medium to the bag, a waste liquid vessel into which the medium is removed from the bag, a pumping means provided in a member connecting the bag and the medium vessel, and a pumping means provided in a member connecting the bag and the waste liquid vessel are provided on the unit, wherein a distance from a bottom surface of the unit to a culture surface is shorter than 20 mm, a liquid thickness of the medium becomes uniform when the pressing member presses the bag such that a surface of the unit, an upper surface and a lower surface of the pressing member as well as an upper surface and a culture surface of the bag become approximately parallel to one another.

## Description

### TECHNICAL FIELD

The invention relates to cell culture technologies, particularly to cell culture systems for culturing and observing cells.

### BACKGROUND ART

In culturing cells in a culture vessel, observing the state of the cells being cultured in the culture vessel with a microscope or a camera is very important for the culturing process. For culturing cells in large quantities, the use of culture bags made of soft packaging material is preferable to easily produce culture vessels with a large culture area.

A technology to enable observation of the state of cells being cultured includes a culture device with a dedicated observation mechanism provided inside a dedicated incubator. Other examples include small culture devices that can be mounted on a general-purpose microscope.

### CITATION LIST

### PATENT DOCUMENTS

Patent Document 1: WO 2005/059091 Pamphlet
Patent Document 2: Japanese Utility Model No. 3115673

### DISCLOSURE OF THE INVENTION

### PROBLEMS TO BE SOLVED BY THE INVENTION

The cell culture device described in Patent document 1 is a culture device with a dedicated observation mechanism provided inside a dedicated incubator.

Since observation operations cannot be performed manually in such a culture device, an observation mechanism that enables automatic operation is required, even for simple operations that are easy if performed manually by an operator such as focusing. This makes the device large and the observation mechanism very expensive.

In addition, separate observation mechanisms for each culture vessel or a mechanism to automatically move the culture vessel or the microscope is required to culture cells using multiple culture vessels and to observe the cells in the culture vessels, which makes the device even more expensive.

Further, when such a culture device uses a culture bag made of soft packaging material, the device tends to cause diffuse reflection of light as it is, making it difficult to observe the cells in the culture bag.

The petri dish described in Patent document 2 is a small culture device that can be mounted on a general-purpose microscope.

Although it is possible to observe cells being cultured in a petri dish according to such a culture device, it is necessary to fix a pipe for removing a medium from or adding the medium onto the lid of the petri dish. Therefore, when the position of the tip of the pipe is located at a distance from the culture surface, the remaining amount of medium at the time of medium removal increases, making it impossible to increase the medium exchange rate.

In addition, when the tip of the pipe is close to the culture surface, cells can be sucked out when removing the medium, and when an uneven culture surface is provided to culture sphere cells, sphere cells can move when adding the medium.

Further, this culture device is not applicable when cells are cultured using a culture bag made of soft packaging material.

Therefore, the inventors have conducted intensive research and succeeded in developing a cell culture system that can solve the above problems.

More specifically, the purpose of the present invention is to provide a cell culture system, a method of using a cell culture system and a cell culture unit that can culture cells by providing a culture bag, can be easily transported with various tools necessary for the culture in one package and can utilize the culture bag for observation with a general-purpose microscope.

### SOLUTIONS TO THE PROBLEMS

To achieve the above-mentioned purpose, a cell culture system of the present invention is a cell culture system for culturing cells using a culture bag made of soft packaging material, including: a culture unit having a cutout portion or a transparent mounting portion on which a bag housing component having a pressing member that presses the culture bag is mounted; and the culture bag placed on the bag housing component, a medium vessel supplying a medium to the culture bag, a waste liquid vessel into which the medium is removed from the culture bag, a first pumping means provided in a first tubular member connecting the culture bag and the medium vessel, and a second pumping means provided in a second tubular member connecting the culture bag and the waste liquid vessel on the culture unit, wherein a distance from a bottom surface of the culture unit to a culture surface of the culture bag is shorter than 20 mm, wherein when the pressing member presses the culture bag filled with the medium to uniformize a liquid thickness of the medium in the culture bag such that a surface of the culture unit, an upper surface and a lower surface of the pressing member, and an upper surface and a culture surface of the culture bag become approximately parallel to one another.

The cell culture system of the present invention is preferably configured such that the culture unit has a first pumping means housing that houses the first pumping means and a second pumping means housing that houses the second pumping means.

The cell culture system of the present invention is also preferably configured such that a distance from a bottom surface of the culture unit to an upper surface of the first pumping means housing and/or an upper surface of the second pumping means housing is 65 mm or shorter.

The cell culture system of the present invention is also preferably configured such that the medium vessel has two ports, and the medium in the medium vessel is allowed to be exchanged via one of the ports while the medium vessel is connected to the culture bag via the other one of the ports.

The cell culture system of the present invention is also preferably configured such that the bag housing component has a liquid thickness measuring unit, the liquid thickness measuring unit is connected to a control device, and the liquid thickness of the medium in the culture bag is calculated by the control device based on a value measured by the liquid thickness measuring unit.

The cell culture system of the present invention is also preferably configured such that the control device is connected to the first pumping means and the second pumping means, and the first pumping means and the second pumping means are controlled by the control device based on the liquid thickness of the medium in the culture bag.

A method of using a cell culture system of the present invention is a method of using a cell culture system according to any of the above, wherein the cell culture system is placed inside an incubator, and the medium is supplied from the medium vessel to the culture bag by the first pumping means to culture cells, wherein the cell culture system is transported out of the incubator and placed on a microscope, and cells in the culture bag are observed using the microscope via the cutout portion or the transparent mounting portion, while the pressing member presses the culture bag such that the liquid thickness of the medium in the culture bag becomes uniform.

The method of using a cell culture system of the present invention is preferably a method of observing cells in the culture bag using the microscope, with the medium being pumped from the medium vessel to the culture bag and with the medium being pumped from the culture bag to the waste liquid vessel.

A cell culture unit of the present invention is a cell culture unit that can culture cells by providing a culture bag, wherein the cell culture unit includes a cutout portion or a transparent mounting portion on which a culture bag housing component housing the culture bag is mounted, and a main body having two pumping means housings on which a pumping means connected to the culture bag via a tubular member is mountable, and a mounting plate on which a medium supply bag connected to one of the pumping means via a tubular member is mountable in a tiltable manner, wherein a waste liquid bag connected to the other pumping means via a tubular member is mountable on the lower side of the mounting plate.

The cell culture unit of the present invention preferably includes the culture bag housing component housing the culture bag, the medium supply bag and the waste liquid bag, wherein the cell culture unit is allowed to be transported with the arrangement of the culture bag, the medium supply bag and the waste liquid bag being maintained.

### EFFECTS OF THE INVENTION

According to the present invention, a cell culture system, a method of using a cell culture system, and a cell culture unit that can culture cells by providing a culture bag can be easily transported with various tools necessary for the culture in one package and can utilize the culture bag for observation with a general-purpose microscope can be provided.

### BRIEF DESCRIPTION OF THE DRAWINGS

FIG. 1 is a schematic diagram (perspective view) illustrating a configuration of a cell culture unit according to an embodiment of the present invention.
FIG. 2 is a schematic diagram (front view) illustrating the configuration of the cell culture unit according to the embodiment of the present invention.
FIG. 3 is a schematic diagram (rear view) illustrating the configuration of the cell culture unit according to the embodiment of the present invention.
FIG. 4 is a schematic diagram (plan view) illustrating the configuration of the cell culture unit according to the embodiment of the present invention.
FIG. 5 is a schematic diagram (bottom view) illustrating the configuration of the cell culture unit according to the embodiment of the present invention.
FIG. 6 is a schematic diagram (right-side view) illustrating the configuration of the cell culture unit according to the embodiment of the present invention.
FIG. 7 is a schematic diagram (left-side view) illustrating the configuration of the cell culture unit according to the embodiment of the present invention.
FIG. 8 is a schematic diagram illustrating the configuration of the cell culture unit (with a culture bag housing component mounted on top) according to the embodiment of the present invention.
FIG. 9 is a schematic diagram illustrating how the cell culture unit according to the embodiment of the present invention is being used.
FIG. 10 is a schematic diagram illustrating the culture bag housing component that can be mounted on the cell culture unit according to the embodiment of the present invention.
FIG. 11 is a schematic diagram illustrating a control mechanism of the cell culture unit according to the embodiment of the present invention.
FIG. 12 is a schematic diagram illustrating a configuration of a cell culture system according to the embodiment of the present invention.
FIG. 13 is a schematic diagram illustrating a distance from a bottom surface of the culture unit to a culture surface of the culture bag (L1) in the cell culture system according to the embodiment of the present invention.
FIG. 14 is a schematic diagram illustrating a distance from the bottom surface of the culture unit to an upper surface of a pumping means housing (L2) in the cell culture system according to the embodiment of the present invention.
FIG. 15 is a schematic diagram illustrating how the cell culture system according to the embodiment of the present invention is placed on a microscope.
FIG. 16 is a schematic diagram illustrating the control mechanism of the cell culture system according to the embodiment of the present invention.

### DESCRIPTION OF PREFERRED EMBODIMENTS

An embodiment of the present invention, such as a cell culture system, a method of using the cell culture system and a cell culture unit, will be described in detail below. Note, however, that the present invention is not limited to the specifics of the following embodiment.

### [Cell culture unit]

First, a cell culture unit of the embodiment will be described. The cell culture unit of the embodiment is a jig on which cells are cultured by providing a culture bag.

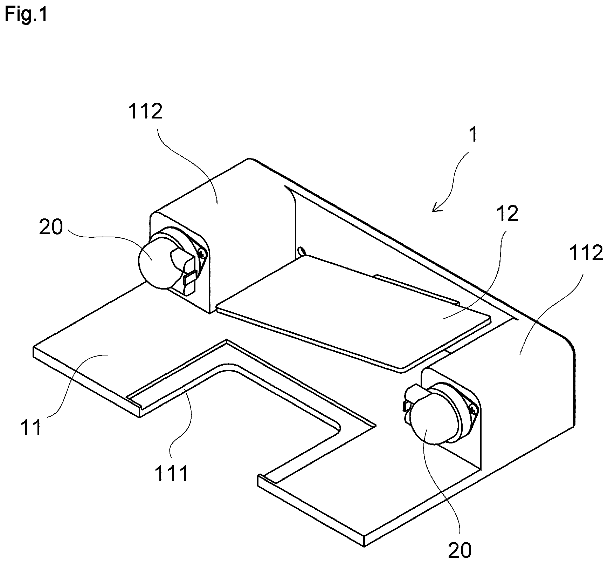

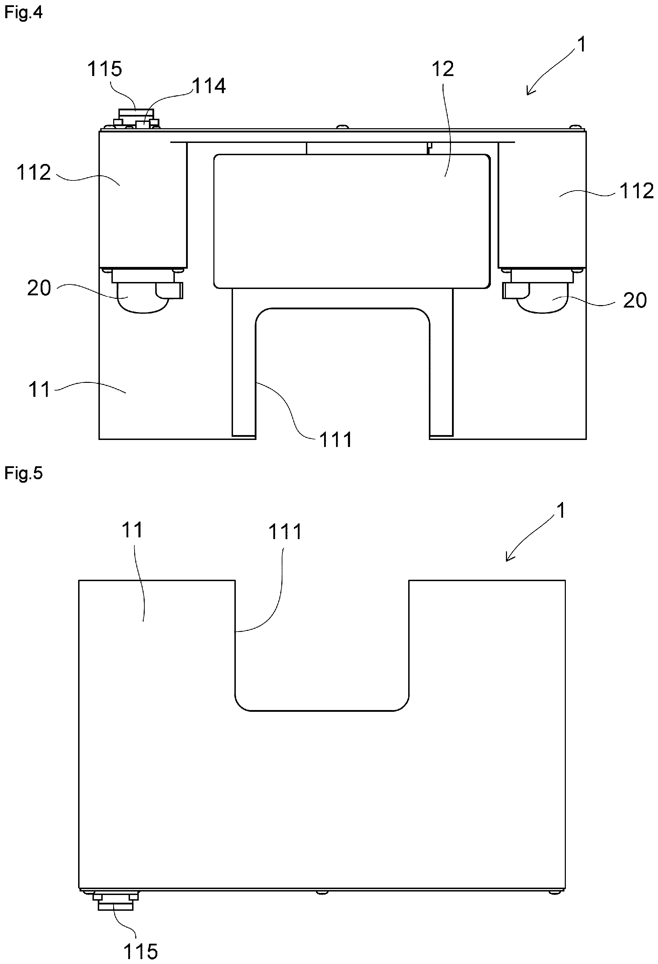

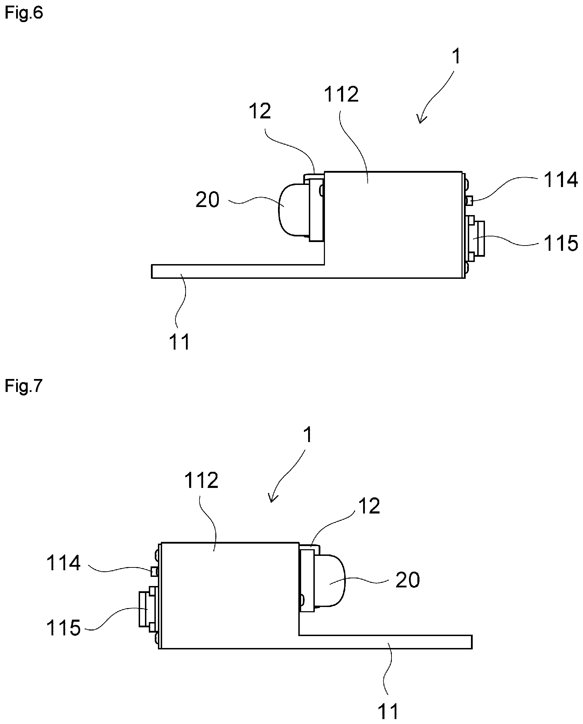

As shown in FIGS. 1-7, the cell culture unit 1 of the embodiment comprises a main body 11 and a mounting plate 12.

The main body 11 has a cutout portion 111 on which a culture bag housing component housing the culture bag is mounted, and two pumping means housings 112 that can house a pumping means, such as a pump 20, that is connected to the culture bag via a tubular member, such as a tube.

A support groove, which can support the culture bag housing component, is formed in the periphery of the cutout portion 111.

A medium supply bag that is connected to one of the pumps 20 via the tubular member can be mounted on the mounting plate 12 in a tiltable manner.

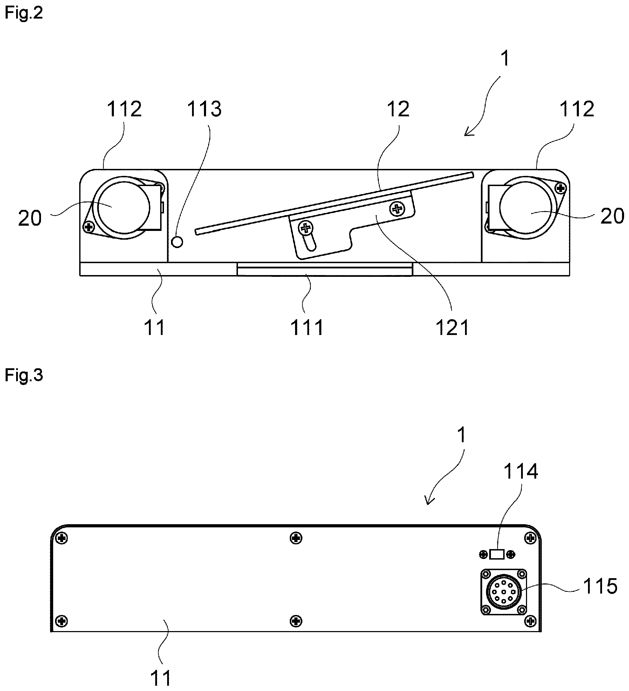

Specifically, as shown in FIG. 2, the mounting plate 12 comprises a mounting plate support portion 121, and the mounting plate support portion 121 enables the tilt angle of the mounting plate 12 to be adjusted between a horizontal state and a tilted state.

It also enables air bubbles in the medium supply bag to be collected at the upper portion to prevent the air bubbles from being sent to the culture bag by mounting the medium supply bag on the tilted mounting plate 12.

A waste liquid bag that is connected to the other one of the pumps 20 via the tubular member can be mounted on the lower side of the mounting plate 12.

Two pumping means housings 112, on which the pumps 20 can be placed, are provided at both ends of the rear side of the main body 11, the culture bag housing component 30 can be mounted in the center portion of the front side of the main body 11, and the mounting plate 12 is provided between the two pumping means housings 112. Therefore, by mounting the medium supply bag 50 on top of the mounting plate 12 and the waste liquid bag 60 on the lower side of the mounting plate 12, these bags can be compactly arranged in the cell culture unit and the various tools necessary for cell culture can be transported in a well-balanced manner.

A sensor cable opening 113 and a sensor cable opening 114 are provided in the main body 11 such that cables (such as signal cables) that are connected to a liquid thickness measuring unit 31, such as a length measuring sensor provided in the culture bag housing component 30, which will be described later, are connected to a control device 70 via these cable openings to enable signals from the liquid thickness measuring unit 31 to be transmitted to the control device 70.

A connector connecting portion 115 is also provided in the main unit 11 such that cables (such as power supply cables) that are connected to the pumps 20 are connected to the control device 70 via a connector that is connected to the connector connecting portion 115 to enable the pumps 20 to be controlled by the control device 70.

The pump 20 is preferably a pump that can pump a liquid at a low flow rate with high accuracy, such as a peristaltic pump or a syringe pump.

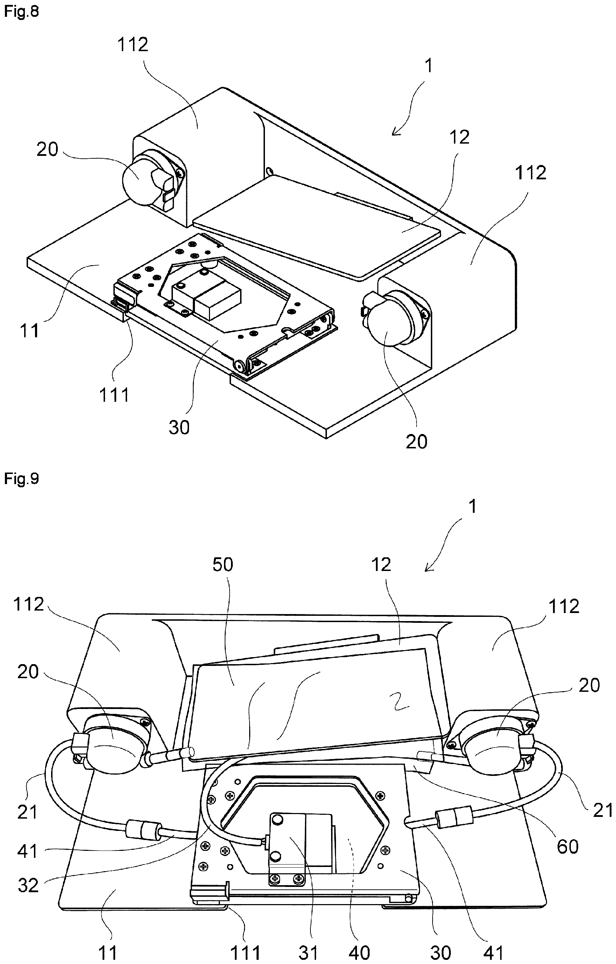

As shown in FIG. 8, a culture bag housing component 30 housing the culture bag can be mounted on the cutout portion 111 of the main body 11.

In this way, by mounting the culture bag housing component 30 on the cutout portion 111, cells in the culture bag can be observed with a microscope from below the culture bag housing component 30.

Instead of this cutout portion 111, a transparent mounting portion can be provided in the main body 11 with a configuration in which the culture bag housing component 30 is placed on this mounting portion to enable cells in the culture bag to be observed from below the culture bag housing component 30.

FIG. 9 shows how the culture bag housing component 30 housing the culture bag 40, the medium supply bag 50 and the waste liquid bag 60 are mounted on the cell culture unit 1 of the embodiment, the culture bag 40 and the medium supply bag 50 are connected via the pump 20 with a tube 21, and the culture bag 40 and the waste liquid bag 60 are connected via the pump 20 with a tube 21. The culture bag housing component 30 comprises the liquid thickness measuring unit 31 and a sensor cable 32 is connected to this liquid thickness measuring unit 31.

Such a cell culture unit can be transported with the arrangement of the culture bag 40, the medium supply bag 50 and the waste liquid bag 60 being maintained.

Culturing cells by stacking cell culture units with such various tools necessary for culture arranged in one package inside an incubator is also preferable.

In this way, the culture area can be easily extended by culturing cells with the cell culture units of the embodiment stacked together inside the incubator.

The culture bag 40 is a bag-shaped, closed system culture vessel made of highly gas-permeable soft packaging material, which is filled with cells and a medium to culture cells.

The material of the culture bag 40 preferably includes a resin film or the like, and polyolefin resins such as polyethylene and polypropylene can be used. Examples include polyethylene, copolymers of ethylene and α-olefins, copolymers of ethylene and vinyl acetate, and ionomers containing copolymers of ethylene and acrylic acid or methacrylic acid, and metal ions. Polyolefins, styrene elastomers, polyester-based thermoplastic elastomers, etc. can also be used. Further, soft vinyl chloride resins, polybutadiene resins, ethylene-vinyl acetate copolymers, chlorinated polyethylene resins, polyurethane thermoplastic elastomers, polyester thermoplastic elastomers, silicone thermoplastic elastomers, styrene elastomers, such as SBS (styrene butadiene styrene), SIS (styrene isoprene styrene), SEBS (styrene ethylene butylene styrene), SEPS (styrene ethylene propylene styrene), polyolefin resins, fluororesins, etc. may be used.

The cells to be cultured using the culture bag 40 are not limited and may be floating cells, adherent cells, spheres (cell clusters) or organoids prepared by aggregating different types of cells. Specifically, examples include nerve cells, pancreatic islet cells, renal cells, hepatic cells, muscle cells, cardiac muscle cells, corneal endothelial cells, vascular endothelial cells, mesenchymal stem cells, and lymphocytes. Examples also include induced pluripotent stem cells (iPS cells), embryonic stem cells (ES cells) and cells differentiated and induced from these stem cells into the above-mentioned various types of cells. Further, intermediates such as progenitor cells that are generated during the process of differentiation induction can also be cultured.

In the embodiment, a port 41 used for supplying the medium and a port 41 used for removing the medium are provided on the culture bag 40. Note that the culture bag 40 may comprise three or more ports.

The medium supply bag 50 is a container filled with the medium to be pumped to the culture bag 40 and is connected to the culture bag 40 via the tube 21 and the pump 20.

The waste liquid bag 60 is a container to which a used medium is pumped from the culture bag 40 and is connected to the culture bag 40 via the tube 21 and the pump 20.

The medium supply bag 50 and the waste liquid bag 60 can be made of the same material as the culture bag 40. Note that the medium supply bag 50 and the waste liquid bag 60 may be a gas-barrier bag with low gas permeability, and the material for these bags is not limited to any particular material.

According to the embodiment, cells can be cultured by placing such a cell culture unit inside a general-purpose incubator, connecting the cable that is connected to the liquid thickness measuring unit 31 and the cable that is connected to the pump 20 to the control device 70, and operating the pump 20 to supply the medium from the medium supply bag 50 to the culture bag 40. The medium in the culture bag 40 can also be removed into the waste liquid bag 60.

After disconnecting the cables from the cell culture unit in which cells have been cultured in the culture bag 40, the cell culture unit is removed from the incubator with the arrangement of the culture bag 40, the medium supply bag 50 and the waste liquid bag 60 maintained, and moved to a general-purpose microscope while maintaining the closed system to enable the cells in the culture bag to be observed with the microscope via a cutout portion 111.

In other words, such a cell culture unit enables cells to be cultured by providing the culture bag 40 made of soft packaging material without the need for a large-scale and expensive observation mechanism, and the various tools necessary for the culture can be easily transported while maintaining the closed system in one package to enable the culture bag 40 to be utilized for observation with the microscope.

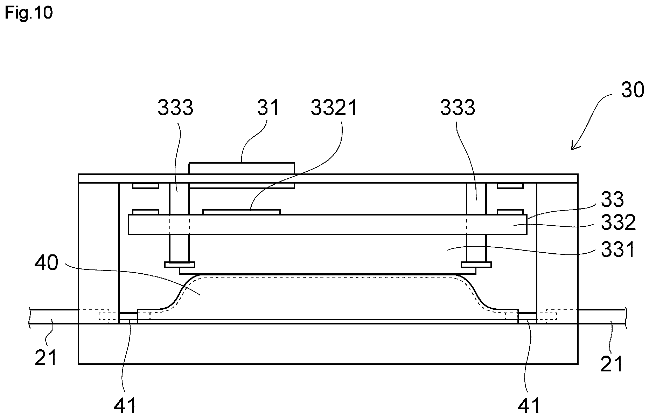

Next, referring to FIG. 10, the configuration of the culture bag housing component 30 housing the culture bag 40 will be described.

A pressing member 33 is provided in the culture bag housing component 30, and the pressing member 33 presses the culture bag 40 filled with the medium to make a liquid thickness of the medium in the culture bag 40 (a distance from an inner surface of an upper film of the culture bag 40 to an inner surface of a lower film (a culture surface)) uniform.

In FIG. 10, the pressing member 33 comprises a pressing portion 331 and a mounting portion 332, with guide pins 333 provided in the periphery of a top plate portion, the guide pins 333 penetrate through guide holes provided in the mounting portion 332, and the pressing member 33 is movable in a vertical direction along the guide pins 333.

The pressing member 33 is movable in a vertical direction while pressing the upper film of the culture bag 40 in a vertical direction due to a permanent magnet or the pressing member 33's own weight.

Specifically, when the culture bag 40 is filled with the medium, the pressing member 33 is pushed up by the culture bag 40, and when the medium is removed from the culture bag 40, the liquid thickness of the medium in the culture bag 40 decreases accordingly, resulting in the pressing member 33 moving downwards.

Using such a culture bag housing component 30, the pressing member 33 can follow and press the upper film of the culture bag 40 in accordance with the liquid volume of the medium in the culture bag 40, enabling the cells in the culture bag 40 to be observed even while the medium is being pumped.

In addition, using such a culture bag housing component 30, the upper and lower films of the culture bag 40 can be prevented from contacting each other even when the liquid thickness of the medium in the culture bag 40, for example, is decreased to 1 mm or thinner.

The pressing member 33 may be made of, for example, a synthetic resin such as polycarbonate, or glass. The pressing member 33 is partly or wholly transparent.
A top plate opening is provided in the top plate portion of the culture bag housing component 30 to enable the inside of the culture bag housing component 30 to be viewed via this top plate opening.

In the cell culture unit in the embodiment, a surface of the main body 11 is approximately parallel to un upper surface and a lower surface of the pressing member 33 as well as un upper surface and the culture surface of the culture bag 40.

This prevents diffuse reflection of light and enables the cells to be preferably observed when the light is irradiated from above the culture bag 40 to observe the cells in the culture bag 40 from below with the microscope.

In the cell culture unit of the embodiment, a distance from a bottom surface of the main body 11 to the culture surface in the culture bag 40 (a thickness of a mounting platform of the main body 11 + a thickness of a mounting base of the culture bag housing component 30 + a thickness of the lower film of the culture bag 40) is preferably shorter than 20 mm, more preferably shorter than 10 mm, and particularly preferably around 5-6 mm.

With the cell culture unit of the embodiment configured in this way, the cells in the culture bag 40 can be preferably observed using a general-purpose phase contrast microscope or the like.

The liquid thickness measuring unit 31 is fixed to the top plate portion of the culture bag housing component 30, and a metal member 3321 is mounted on the mounting portion 332 of the pressing member 33 such that it faces the measuring unit 31.

A distance to the metal member 3321 is measured by the liquid thickness measuring unit 31 and output to the control device 70 such that the control device 70 can calculate the liquid thickness of the medium in the culture bag 40.

The pumps 20 are controlled by the control device 70 based on the liquid thickness of the medium in the culture bag 40.

Specifically, the control device 70 calculates the liquid thickness of the medium in the culture bag 40 based on the value (output voltage or output current) measured by the liquid thickness measuring unit 31 and controls the start and stop of the operation of the pumps 20 as well as the rotation speed, etc. based on the liquid thickness to control the liquid thickness to a desired level.

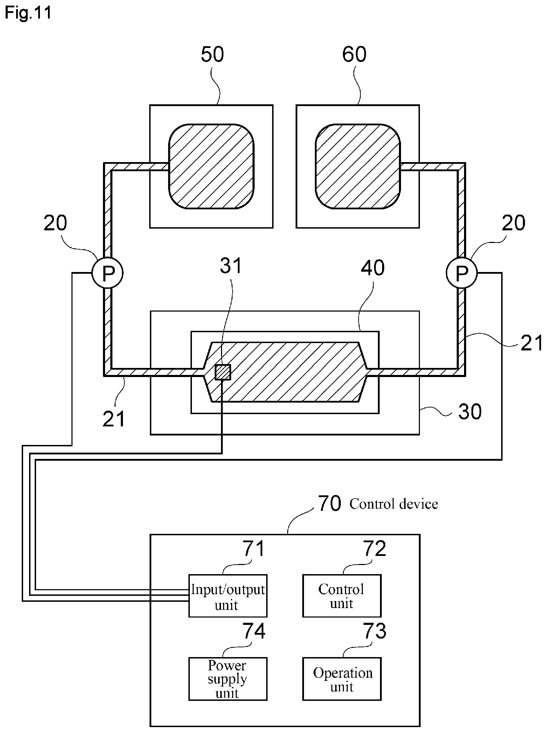

As shown in FIG. 11, the control device 70 can have an input/output unit 71, a control unit 72, an operation unit 73 and a power supply unit 74.

The input/output unit 71 is connected to the pumps 20 and controls the operation of the pumps 20 based on input information from the control unit 72. This enables the medium to be supplied to the culture bag 40 from the medium supply bag 50 and the used medium to be removed from the culture bag 40 into the waste liquid bag 60.

The control unit 72 comprises a programmable logic controller (PLC) or the like, in which the intended control details are pre-programmed and stored to control the operation of each part based on these details. Specifically, the control unit 72 transmits information to control the pumps to the input/output unit 71 at specific timings.

A display unit, such as a touch panel, is provided in the operation unit 73, which transmits information input by the user to the control unit 72 to execute settings of the PLC, etc. It displays the input information from the control unit 72 at the same time.

The power supply unit 74 (such as a regulated power supply) supplies electricity to each unit in the control unit 70.

Although not shown in the figure, the control device 70 can further comprise a relay or a circuit breaker. Part or all of the control unit 72, the operation unit 73 and other units in the control device 70 may be configured with a microcontroller or a computer.

### [Method of using cell culture unit]

The cell culture unit of the embodiment is used for culturing cells by placing the cell culture unit described above inside the incubator and supplying the medium from the medium supply bag 50 to the culture bag 40 via the tube 21 using one of the pumps 20.

The medium can also be removed from the culture bag 40 into the waste liquid bag 60 via the tube 21 using the other one of the pumps 20.

The cell culture unit is transported out of the incubator with the arrangement of the culture bag 40, the medium supply bag 50 and the waste liquid bag 60 maintained to enable the culture bag 40 to be utilized for cell observation with the microscope via the cutout portion 111 or the transparent mounting portion.

Such a method of using a cell culture unit enables cells to be cultured by placing the cell culture unit inside a general-purpose incubator without the need for a large-scale and expensive observation mechanism. The cell culture unit can be removed from the incubator and easily transported while maintaining the closed system, and can be placed on a general-purpose microscope to observe cells in the culture bag.

### [Cell culture system]

Next, a cell culture system of the embodiment will be described. The cell culture system of the embodiment comprises the cell culture unit and various tools necessary for cell culture described above and has features that can be transported while the cells are being cultured in the culture bag and can be preferably used for observation of the cells in the culture bag with a microscope.

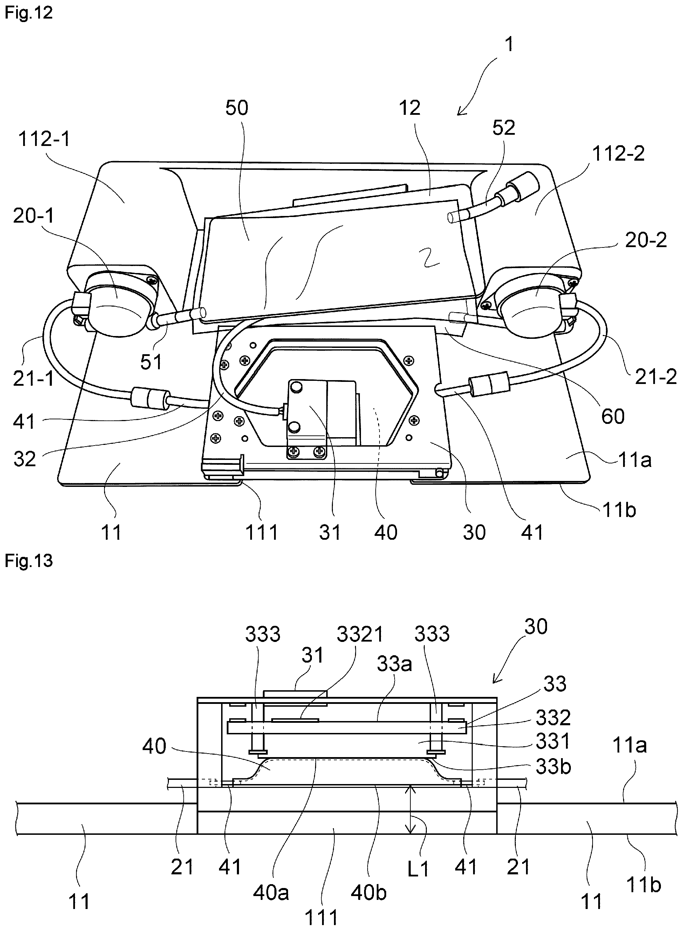

Specifically, as shown in FIG. 12, the cell culture system of the embodiment is a cell culture system for culturing cells using the culture bag 40 made of soft packaging material and comprises a cell culture unit 1 that has a cutout portion 111 or a transparent mounting portion on which the culture bag housing component 30 comprising the pressing member that presses the culture bag 40 is mounted.

The culture bag 40 placed on the culture bag housing component 30, the medium vessel 50 supplying the medium to the culture bag 40, the waste liquid vessel 60 into which the medium is removed from the culture bag 40, a first pumping means 20-1 provided in a first tubular member 21-1 connecting the culture bag 40 and the medium vessel 50, and a second pumping means 20-2 provided in a second tubular member 21-2 connecting the culture bag 40 and the waste liquid vessel 60 are provided on the culture unit 1.

Note that the medium vessel 50 and the waste liquid vessel 60 may be a bag made of soft packaging material or a rigid container.

In the cell culture system of the embodiment, a distance from a bottom surface (11b) of the cell culture unit 1 to the culture surface (40b) of the culture bag 40, L1 (a thickness of a mounting platform of the main body 11 + a thickness of a mounting base of the culture bag housing component 30 + a thickness of the lower film of the culture bag 40), as shown in FIG. 13, is preferably shorter than 20 mm, more preferably shorter than 10 mm, and even more preferably 7-8 mm, and particularly preferably 5-6 mm.

With the cell culture system of the embodiment configured in this way, when the cell culture system is placed on a general-purpose phase contrast microscope or the like, the focal distance can be easily and appropriately adjusted, and the cells in the culture bag 40 can be preferably observed using the microscope.

In the cell culture system of the embodiment, as shown in FIG. 13, the liquid thickness of the medium in the culture bag 40 becomes uniform when the pressing member 33 presses the culture bag 40 filled with the medium such that a surface (11a) of the cell culture unit 1, the upper surface (33a) and the lower surface (33b) of the pressing member 33 as well as the upper surface (40a) and the culture surface (40b) of the culture bag 40 become approximately parallel to one another.

This prevents diffuse reflection of light and enables the cells to be preferably observed when the light is irradiated from above the culture bag 40 to observe the cells in the culture bag 40 from below with the microscope.

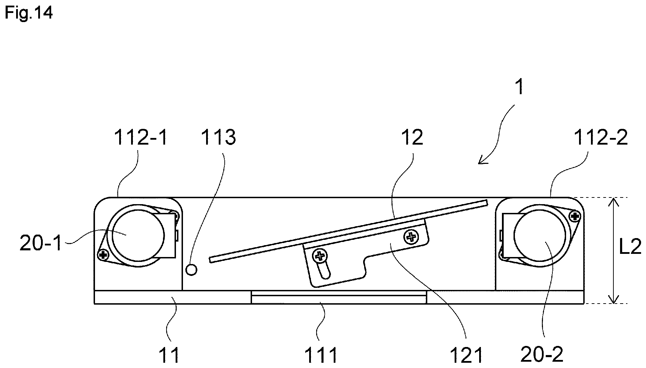

In the cell culture system of the embodiment, as shown in FIG. 14, the cell culture unit 1 has a first pumping means housing 112-1 that houses the first pumping means 20-1 and the second pumping means housing112-2 that houses the second pumping means 20-2.

A distance from the bottom surface of the cell culture unit 1 to un upper surface of the first pumping means housing and/or un upper surface of the second pumping means housing, L2, is preferably 65 mm or shorter, more preferably 60 mm or shorter, and even more preferably 55 mm or shorter.

A general-purpose phase contrast microscope generally has a lens and a light source in the light source unit, which are arranged such that the light converges on the target of observation, thus the light source unit is fixed and cannot be moved up or down. When the cell culture system is placed on the microscope, the height of at least one of the pumping means housings is preferably smaller than the distance between the light source unit and the observation platform, as the system is required to travel under the light source unit.

Therefore, the cell culture system of the embodiment can be easily and appropriately placed on the observation platform without being interfered with by the light source unit of the microscope when the cell culture system is placed on a general-purpose phase contrast microscope or the like, by the above configuration, enabling the cells in the culture bag 40 to be preferably observed using the microscope.

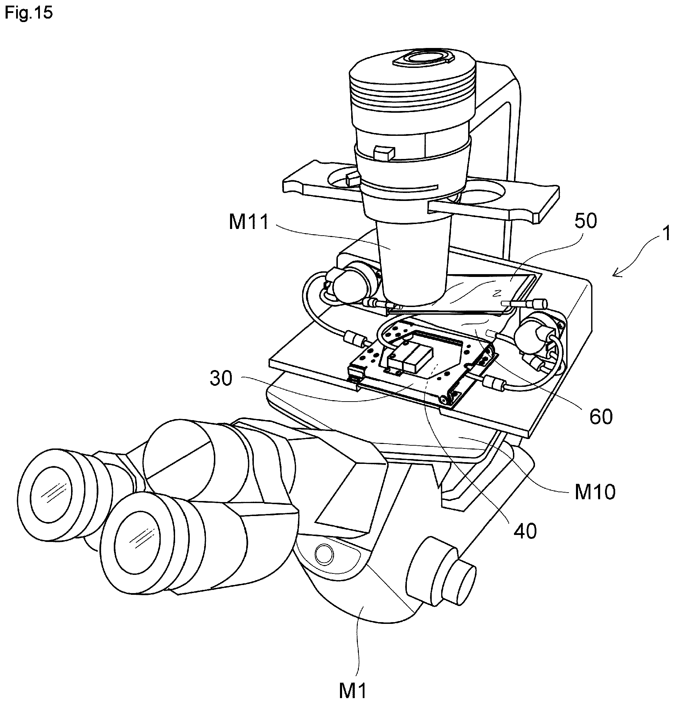

FIG. 15 shows how the cell culture system of the embodiment is placed on a general-purpose phase contrast microscope.

Such a cell culture system enables the cells in the culture bag 40 to be easily observed by being transported while culturing cells with the culture bag 40 and mounted on the observation platform M10 of the general-purpose phase contrast microscope M1. In this case, the cell culture system can be easily placed on the observation platform M10 without being interfered with by the light source unit M11 of the general-purpose phase contrast microscope M1.

On one hand, when observing cells in a culture bag, cells in the culture bag conventionally needed to be observed by placing a bag housing component housing the culture bag, a medium vessel, a waste liquid vessel, two pumping means and tubes connecting them on a platform and transporting them to a microscope, which was very troublesome.

On the other hand, the cell culture system of the embodiment makes it possible to significantly reduce the time and effort required to observe the state of cells when culturing cells using a culture bag. This reduction in time and effort is extremely important for the field of culturing, where the same work needs to be done repeatedly.

In addition, the cell culture system of the embodiment enables the state of the cells being cultured to be observed as it is and also enables the state of the cells to be observed while the medium is being pumped, as described in the method of using the cell culture system below. In this way, the cell culture system of the embodiment enables the observation method that was not easily possible in the past to be easily achieved.

The cell culture system of the embodiment, as shown in FIG. 12, is preferably configured such that the medium vessel 50 has two ports, and the medium in the medium vessel 50 can be exchanged via one of the ports while the medium vessel 50 is connected to the culture bag 40 via the other one of the ports.

Specifically, differentiating and inducing spheres of iPS cells using the cell culture system of the embodiment requires frequent exchange of the types of media. The medium may be exchanged daily, depending on the type of differentiation induction.

In this case, the cell culture system is carried into a clean bench, etc., and the medium in the medium vessel 50 is entirely exchanged, whereas the air inside the clean bench is clean but the system must not make contact with things because of the risk of contamination in case of contact, and thus the work must be done over again when it has made contact.

In the cell culture system of the embodiment, as shown in FIG. 12, the medium vessel 50 has the ports 51 and 52, enabling the medium to be exchanged by opening the port 51 that is connected to the culture bag 40 and the port 52 on the opposite side to connect a syringe or the like. In this way, the cell culture system of the embodiment enables easy operation and reduces the risk of contamination because only one portion, the port 52 of the medium vessel 50, needs to be opened.

On one hand, in the cell culture system of the embodiment, if a medium vessel 50 with only one port 51 to be connected to the culture bag 40 is used, the tube needs to be disconnected near the pump 20-1 to exchange the medium, which means that the tube may make contact with things.

In addition, the two portions of the tube on the side of the culture bag 40 and the port 51 on the side of the medium vessel 50 are opened, and the work has to be done while holding the tube and the port 51 at the same time, which increases the risk of contamination. Further, it is very troublesome because the work involves reaching into a tight space.

On the other hand, the cell culture system of the embodiment, in which the medium vessel 50 comprises two ports, enables easy exchange of the medium in the medium vessel 50, thereby simplifying the culturing process.

In the cell culture system of the embodiment, two ports are also preferably provided for the waste liquid vessel 60 from the point of view of reducing the risk of contamination. Of course, using a large waste liquid vessel 60 may eliminate the need of removing the medium from the waste liquid vessel 60.

The cell culture system of the embodiment preferably comprises a control device as well as a cell culture unit as described above.

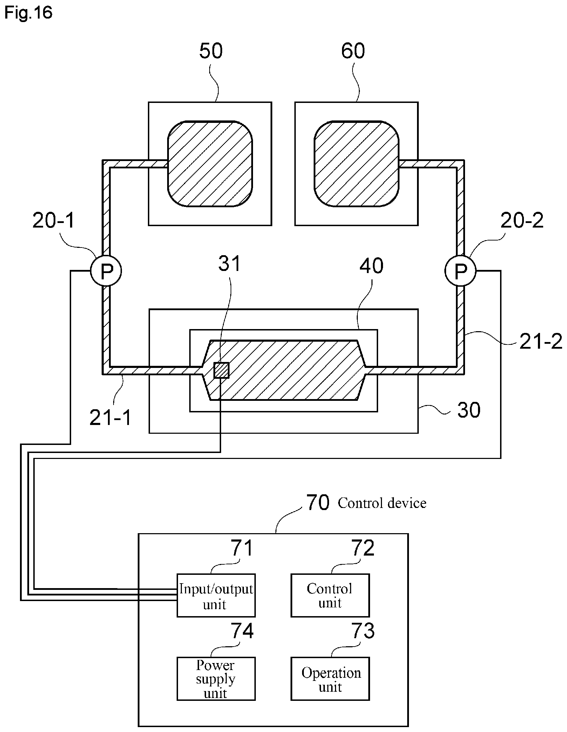

Specifically, as shown in FIG. 16, in the cell culture system of the embodiment, the culture bag housing component 30 has the liquid thickness measuring unit 31, the liquid thickness measuring unit 31 is connected to the control device 70, and the liquid thickness of the medium in the culture bag 40 is calculated by the control device 70 based on the value measured by the liquid thickness measuring unit 31.

A control device 70 is connected to the pump 20-1 and the pump 20-2, and the pump 20-1 and the pump 20-2 are controlled by the control device 70 based on the liquid thickness of the medium in the culture bag 40.

The specific configuration of the control device 70 is omitted here as it has been described above in the description of the cell culture unit.

### [Method of using cell culture system]

The method of using the cell culture system of the embodiment is a method of using the cell culture system described above, wherein the cell culture system is placed inside the incubator, and the medium is supplied from the medium vessel 50 to the culture bag 40 by the pump 20-1 to culture cells, wherein the cell culture system is transported out of the incubator and placed on the microscope M1, and the cells in the culture bag 40 are observed using the microscope M1 via the cutout portion 111 or the transparent mounting portion, while the pressing member 33 presses the culture bag 40 such that the liquid thickness of the medium in the culture bag 40 becomes uniform.

The method of using the cell culture system of the embodiment is also preferably a method of observing the cells in the culture bag 40 using the microscope M1, with the medium being pumped from the medium vessel 50 to the culture bag 40 and with the medium being pumped from the culture bag 40 to the waste liquid vessel 60.

When transporting the cell culture system out of the incubator, the cables that are connected to the liquid thickness measuring unit 31 and the cables that are connected to the pumps 20-1 and 20-2, both of which are connected to the cell culture unit 1, are disconnected, the cell culture system is mounted on the microscope M1, and then the cables are connected again to the cell culture unit 1.

By operating the pumps 20-1 and 20-2, the state of the cells being pumped can be observed with the microscope M1.

Such a method of using the cell culture system of the embodiment enables, for example, the behavior of the spheres, such as whether or not they move as a result of pumping, to be observed, which not only enables the state of the cells being cultured to be monitored but also helps to develop better culture bags and identify the pumping conditions to prevent the spheres from moving.

As described above, the cell culture system, the method of using the cell culture system and the cell culture unit of the embodiment enables cells to be cultured by placing the cell culture unit inside a general-purpose incubator without the need for a large-scale and expensive observation mechanism. The cell culture unit can be removed from the incubator and easily transported while maintaining the closed system, and can be placed on a general-purpose microscope to observe cells in the culture bag.

When the cell culture system is placed on a general-purpose microscope or the like, the focal distance can be easily and appropriately adjusted, diffuse reflection of light can be prevented, and the system can easily and appropriately be placed on the observation platform, enabling the cells in the culture bag to be preferably observed.

Further, the work of observing the state of the cells can be done with much less time and effort, the operation of exchanging the medium is easy, and the risk of contamination can also be reduced.

The state of the cells being cultured can also be observed while the medium is being pumped, and the behavior of the spheres, such as whether or not they move as a result of pumping, can be observed, which not only enables the state of the cells being cultured to be monitored but also helps to develop better culture bags, etc.

Note that, in addition to the above-mentioned configurations, the cell culture unit of the present invention is also preferably configured as follows.

Specifically, the cell culture unit of the present invention is also preferably configured such that cells in the culture bag can be observed via the cutout portion or the transparent mounting portion.

The cell culture unit of the present invention is also preferably configured such that the culture bag housing component comprises a pressing member, and the pressing member presses the culture bag filled with a medium to make a liquid thickness of the medium in the culture bag uniform.

The cell culture unit of the present invention is also preferably configured such that a surface of the main body, un upper surface and a lower surface of the pressing member as well as un upper surface and a culture surface of the culture bag are approximately parallel to one another.

The cell culture unit of the present invention is also preferably configured such that a distance from a bottom surface of the main body to the culture surface in the culture bag is shorter than 20 mm.

The cell culture unit of the present invention is also preferably configured such that the culture bag housing component has a liquid thickness measuring unit, the liquid thickness measuring unit is connected to a control device, and the liquid thickness of the medium in the culture bag is calculated by the control device based on a value measured by the liquid thickness measuring unit.

The cell culture unit of the present invention is also preferably configured such that the control device is connected to the pumping means and the pumping means is controlled by the control device based on the liquid thickness of a medium in the culture bag.

A method of using a cell culture unit of the present invention is a method of using a cell culture unit according to any of the above, wherein the cell culture unit is placed inside an incubator, and the medium is supplied from the medium supply bag to the culture bag via a tubular member using one of the pumping means to culture cells, wherein the medium can be removed from the culture bag into the waste liquid bag via a tubular member using the other one of the pumping means, and the cell culture unit is transported out of the incubator with the arrangement of the culture bag, the medium supply bag and the waste liquid bag being maintained to enable the culture bag to be utilized for cell observation with a microscope via the cutout portion or the transparent mounting portion.

The present invention is not limited to the above embodiment, and needless to say, various modifications and implementations are possible within the scope of the present invention. For example, the arrangement of the pumping means housing, the mounting plate and the cutout portion in the cell culture unit can be changed as appropriate.

### INDUSTRIAL APPLICABILITY

The present invention can be preferably used when various tools necessary for cell culture are compactly packed in a single package, and the tools are carried around and used for microscopic observation of cultured cells.

All references in this specification and the specification of the Japanese application that is the basis of the Paris priority of this application are hereby incorporated by reference.

### DESCRIPTION OF REFERENCE SIGNS

- 1: Cell culture unit
- 11: Main body
- 111: Cutout portion
- 112, 112-1, 112-2: Pumping means housing
- 113: Sensor cable opening
- 114: Sensor cable opening
- 115: Connector connecting portion
- 12: Mounting plate
- 121: Mounting plate support portion

- 20, 20-1, 20-2: Pump (pumping means)
- 21, 21-1, 21-2: Tube (tubular member)

- 30: Culture bag housing component (bag housing component)
- 31: Liquid thickness measuring unit
- 32: Sensor cable
- 33: Pressing member
- 331: Pressing portion
- 332: Mounting portion
- 3321: Metal member
- 333: Guide pin
- 40: Culture bag
- 41: Port
- 50: Medium supply bag (medium vessel)
- 51,52: Port
- 60: Waste liquid bag (waste liquid vessel)
- 70: Control device
- 71: Input/output unit
- 72: Control unit
- 73: Operation unit
- 74: Power supply unit

## Claims

1. A cell culture system for culturing cells using a culture bag made of soft packaging material, including:
a culture unit having a cutout portion or a transparent mounting portion on which a bag housing component having a pressing member that presses the culture bag is mounted; and
the culture bag placed on the bag housing component, a medium vessel supplying a medium to the culture bag, a waste liquid vessel into which the medium is removed from the culture bag, first pumping means provided in a first tubular member connecting the culture bag and the medium vessel, and second pumping means provided in a second tubular member connecting the culture bag and the waste liquid vessel, on the culture unit,
wherein a distance from a bottom surface of the culture unit to a culture surface of the culture bag is shorter than 20 mm,
wherein the pressing member presses the culture bag filled with the medium to uniformize a liquid thickness of the medium in the culture bag such that a surface of the culture unit, an upper surface and a lower surface of the pressing member, and an upper surface and the culture surface of the culture bag become approximately parallel to one another.

2. The cell culture system according to claim 1, wherein the culture unit has a first pumping means housing that houses the first pumping means and a second pumping means housing that houses the second pumping means.

3. The cell culture system according to claim 2, wherein a distance from the bottom surface of the culture unit to an upper surface of the first pumping means housing and/or an upper surface of the second pumping means housing is 65 mm or shorter.

4. The cell culture system according to any one of claims 1 to 3, wherein the medium vessel has two ports, and the medium in the medium vessel is allowed to be exchanged via one of the ports while the medium vessel is connected to the culture bag via the other one of the ports.

5. The cell culture system according to any one of claims 1 to 4, wherein the bag housing component has a liquid thickness measuring unit, the liquid thickness measuring unit is connected to a control device, and the liquid thickness of the medium in the culture bag is calculated by the control device based on a value measured by the liquid thickness measuring unit.

6. The cell culture system according to claim 5, wherein
the control device is connected to the first pumping means and the second pumping means, and
the first pumping means and the second pumping means are controlled by the control device based on the liquid thickness of the medium in the culture bag.

7. A method of using the cell culture system according to any one of claims 1 to 6,
wherein the cell culture system is placed inside an incubator, and the medium is supplied from the medium vessel to the culture bag by the first pumping means to culture cells,
wherein the cell culture system is transported out of the incubator and placed on a microscope, and cells in the culture bag are observed using the microscope via the cutout portion or the transparent mounting portion, while the pressing member presses the culture bag to uniformize the liquid thickness of the medium in the culture bag becomes uniform.

8. The method of using the cell culture system according to claim 7, wherein cells in the culture bag are observed using the microscope, with the medium being pumped from the medium vessel to the culture bag and with the medium being pumped from the culture bag to the waste liquid vessel.

9. A cell culture unit on which cells are cultured by providing a culture bag, comprising
a cutout portion or a transparent mounting portion on which a culture bag housing component housing the culture bag is mounted; a main body having two pumping means housings on which a pumping means connected to the culture bag via a tubular member is mountable; and a mounting plate on which a medium supply bag connected to one of the pumping means via a tubular member is mountable in a tiltable manner, wherein a waste liquid bag connected to the other pumping means via a tubular member is mountable on the lower side of the mounting plate.

10. The cell culture unit according to claim 9, comprising
the culture bag housing component housing the culture bag, the medium supply bag, and the waste liquid bag,
wherein the cell culture unit is allowed to be transported with the arrangement of the culture bag, the medium supply bag, and the waste liquid bag being maintained.
